# EUROPEAN PATENT SPECIFICATION

(11) **EP 1 497 323 B1**
(45) Date of publication and mention of the grant of the patent: **21.01.2009**
(21) Application number: 03749859.9
(22) Date of filing: 22.04.2003
(51) Int. Cl.: C07K 14/16, A61K 38/16, A61K 9/127

(54) **MULTIPLE BRANCH PEPTIDE CONSTRUCTIONS COMPRISING FROM 2 TO 16 BRANCHES OF THE PEPTIDE RQGYS OR FROM 8 TO 16 BRANCHES OF THE PEPTIDE RQGY PROVIDING FROM THE HIV GP41 GLYCOPROTEIN**
MEHRFACH VERZWEIGTE PEPTIDKONSTRUKTION MIT 2 BIS 16 VERZWEIGUNGEN DES PEPTIDS RQGYS ODER 8 BIS 16 VERZWEIGUNGEN DES PEPTIDS RQGY AUS DEM HIV GP41 GLYCOPROTEIN
CONSTRUCTIONS PEPTIDIQUES A RAMIFICATIONS MULTIPLES COMPRENANT ENTRE 2 ET 16 RAMIFICATIONS DU PEPTIDE RQGYS OU ENTRE 8 ET 16 RAMIFICATIONS DU PEPTIDE RQGY PROVENANT DE LA GLYCOPROTEINE GP41 DU VIH

(30) Priority: 19.04.2002 US 126915
(43) Date of publication of application: 19.01.2005
(73) Proprietor: Cellpep Pharma Inc., Verdun, QC H3E 1H4 (CA)
(72) Inventor: MABROUK, Kamel, F-13003 Marseille (FR); SABATIER, Jean-Marc, F-13790 Rousset (FR); ROCHAT, Hervé, F-13105 Mimet (FR); VAN RIETSCHOTEN, Jurphaas, F-13100 Aix-en-Provence (FR)
(74) Representative: Serjeants
(86) International application number: PCT/EP2003/004353
(87) International publication number: WO 2003/095479

(56) References cited:
- WO-A-98/29443
- WO-A-99/34777
- US-A- 5 622 933
- SABATIER J M ET AL: "Anti-HIV activity of multibranched peptide constructs derived either from the cleavage sequence or from the transmembrane domain (gp41) of the human immunodeficiency virus type 1 envelope." VIROLOGY. UNITED STATES 15 SEP 1996, vol. 223, no. 2, 15 September 1996 (1996-09-15), pages 406-408, XP002253691 ISSN: 0042-6822
- YAHI N ET AL: "MULTIBRANCHED V3 PEPTIDES INHIBIT HUMAN IMMUNODEFICIENCY VIRUS INFECTION IN HUMAN LYMPHOCYTES AND MACROPHAGES" JOURNAL OF VIROLOGY, THE AMERICAN SOCIETY FOR MICROBIOLOGY, US, vol. 68, no. 9, 1 September 1994 (1994-09-01), pages 5714-5720, XP002063190 ISSN: 0022-538X
- TAM J P: "SYNTHETIC PEPTIDE VACCINE DESIGN: SYNTHESIS AND PROPERTIES OF A HIGH-DENSITY MULTIPLE ANTIGENIC PEPTIDE SYSTEM" PROCEEDINGS OF THE NATIONAL ACADEMY OF SCIENCES OF USA, NATIONAL ACADEMY OF SCIENCE. WASHINGTON, US, vol. 85, 1 August 1988 (1988-08-01), pages 5409-5413, XP002070407 ISSN: 0027-8424 cited in the application

## Description

The invention relates to multiple branch peptide constructions (MBPCs) and to their use in the treatment of Human Immunodeficiency Virus (HIV) infections.

The use of radially branched systems in polymers has been known for a long time in classical polymer chemistry. This system has been used by J.P.Tam [Proc. Natl. Acad. Sci. USA 85, 5409-5413 (1988)] to develop antigens without the use of ambiguous carriers, using lysine skeletons. Those antigens were designed to generate vaccines against a variety of diseases. Antigens for generating vaccines against HIV infection are described by Tam in WO93/03766. He called them Multiple Antigenic Peptide Systems (MAPS), consistent with their conceived use.

The present inventors, along with others, found that similar constructions with shorter peptides derived from the V3 loop of the surface envelope glycoprotein gp120 of HIV offered a direct therapeutic approach to the treatment of HIV infections, as described in WO95/07929. The name MAPS was then inappropriate, and the compounds were renamed as MBPCs. The MBPCs of WO95/07929 interfered with the virus envelope - cell membrane fusion step and also the infected cell membrane - uninfected cell membrane fusion step, either step being thought to be indispensable for cell infection, virus multiplication and the spread of virus in the host organism, by blockading the CD4 receptor present in cells such as lymphocytes and macrophages, apparently by attaching to a membrane co-receptor which is distinct from the CD4 binding receptor, without causing the cell to lose its ability to be activated by other antigens or mitogens.

Subsequently the inventors discovered further MBPCs which are effective as treatments for HIV infections. These further MBPCs incorporated peptides derived from the HIV envelope transmembrane glycoprotein gp41, and were described in WO 98/29443. They comprised a core matrix to which are bonded from 2 to 16, and preferably from 4 to 16 peptides, each of which comprises the sequence RQGY preceded by from 0 to 4 amino acid residues and succeeded by from 2 to 4 amino acid residues, but not including more than 10 amino acids. The preferred MBPC in WO 98/29443 had 8 branches of the peptide RQGYSPL on a lysine core matrix. Subsequently to the invention described in WO 98/29443, it was been established that the MBPC [RQGYSPL]₂-K-βA is effective but that the MBPC [RQGYSP]₂-K-βA is less so. This was thought to confirm the lower limit of 6 amino acids in the peptide branches of the MBPCs.

It has now most surprisingly been found that certain short MBPCs retain the effectiveness of the compounds of WO 98/29443. Accordingly, the invention provides a multiple branch peptide construction comprising a core matrix to which are bonded from 2 to 16 peptides, each of which comprises the amino acid sequence RQGYS. The invention further provides a multiple branch peptide construction comprising a core matrix to which are bonded 8 or 16 peptides, each of which comprises the amino acid sequence RQGY. In jurisdictions in which methods for treatment of the human or animal body by therapy are patentable, the invention also provides a method for the therapeutic treatment of patients with HIV infections, the method comprising administering such an MBPC to the patient, preferably in such an amount as to induce in the patient a blood concentration of the MBPC of from 10⁻⁷ to 10⁻⁴ molar.

The core matrix is a dendritic polymer which is branched in nature, preferably with each of the branches thereof being identical. The core matrix is based on a core molecule which has at least two functional groups to which molecular branches having terminal functional groups are covalently bonded. Suitable core molecules include ammonia or ethylenediamine. Suitable molecular branches include acrylic ester monomers which are polymerized onto the core molecule. Such molecules may be created to present varying number of branches, depending on the number of monomers branched from the core molecule. The preferred core molecule is lysine. A central lysine residue is bonded to two lysine residues, each through its carboxyl group, to one of the amino groups of the central lysine residue. This provides a molecule with four amino groups, which may be the core matrix for an MBPC having four peptides. Alternatively, one can provide a molecule with eight branches by bonding four lysine residues through their carboxyl groups to one of the amino groups of the lysine residues which are attached to the central lysine. This molecule can serve as the core matrix for an MBPC having eight peptides or can alternatively receive eight lysine residues to form a core matrix for an MBPC having sixteen peptides.

The C-ends of peptides are covalently bonded to each of the branches of the core matrix to form the MBPC. The resulting molecule has a cluster of peptides at the surface and an interior core matrix which is not presented and is therefore not antigenic.

Spacers may, if desired, be included between the peptides and the core matrix. The carboxyl group of the first lysine residue may be left free, amidated, or coupled to β-alanine or another blocking compound.

Peptides can include D or L-amino acid residues. D amino acids last longer in vivo because they are harder for peptidase to cut, but the L amino acids have better activity, as discussed below.

Moreover, peptide analogues, synthetic constructs using the carbon skeleton of peptides but omitting the -CONH- peptide bonds, can be employed in place of peptides. Thus, it should be understood that references to peptides herein may also be taken to include peptide analogues. It is believed that peptide analogues will be more resistant to peptidase and last longer in vivo.

The preferred MBPCs for use in this invention are
Short RL: [RQGYS]₂-K-βA-OH and
RL3: [RQGY]₈-(K)₄-(K)₂-K- βA-OH

The OH terminal shown above on the β-alanine indicates the carboxyl group thereof, with the amino group being attached to the carboxyl group of the lysine residue. The carboxyl group of the β-alanine may alternatively be modified to form a carboxamide terminal.

Short RL has 2 peptides of 5 amino acids each as compared to 8 peptides of 7 amino acids each in [RQGYSPL]₈-(K)₄-(K)₂-K-βA-OH (RL1, the preferred compound of WO 98/29443). Short RL will therefore be much cheaper to make. As it has an efficacy similar to that of RL1, it represents a significant advance.

WO 99/34777 describes the benefits of liposomes which have a sufficient size for white blood cell internalisation and contain MBPCs useful for the treatment of HIV. Such liposomes may have an average size of greater that 150 nm, and preferably of from 250 nm to 400 nm. They desirably contain above 8% by weight of the MBPC. They make the encapsulated MBPCs more available to the lymphatic system and to the lymphocytes and macrophages which are the target cells for HIV and therefore for anti-HIV substances. It will be understood that the MBPCs of this invention may be contained in such liposomes.

The preparation of the MBPCs of the invention, having a branched core with peptides attached thereto, can be effected by methods known in the art, see e.g. Tam et al, J. Immun. 148, 914-920 (1992). Preferably, for small quantities (under one kilogram), a solid phase method is used to obtain the MBPCs. Stepwise assembly of the peptide chains can be carried out automatically on 4-(oxymethyl)-phenylacetamidomethyl copoly(styrene-1% divinyl benzene). The Boc/benzyl strategy may be used, including a systematic double coupling scheme with hydroxybenzotriazole active esters (Boc-amino-acid-OBt). The final cleaving from resin is effected with strong acid, such as anhydrous hydrogen fluoride (1 hour at 0øC). The MBPC is then washed withdiethyl ether and solubilized in water. After lyophilization, the MBPC may be pre-purified on a P2 or G15 type molecular filtration column, equilibrated with 0.1N acetic acid. The eluate fraction may then be recovered. The purification step is achieved by using C8 or C18 reversed-phase HPLC. The MBPC may be characterized by its amino acid content after acid hydrolysis (6N HCl, 115°C, 24 hours) and electrospray mass spectrometry.

### EXPERIMENTAL SECTION

### Materials

N-α-fluorenylmethyloxycarbonyl (Fmoc) amino acid derivatives were purchased from Perkin-Elmer. All solvents were analytical-grade commercial products from Perkin-Elmer or SDS (Peypin, France).

Human PBLs obtained from healthy HIV-seronegative donor (Etablissement Français du Sang, Marseille, France) were isolated by ficoll-Hypaque gradient centrifugation. Cells were cultured in R10 medium supplemented with 20 units/ml of interleukin-2 (IL-2, Proleukin, Chiron, The Netherlands). R10 medium consists of RPMI 1640 supplemented with 2 mM ultraglutamine (Bio Whittaker, Verviers, Belgium), penicillin (100 units/ml), streptomycin (100 µg/ml), and 10 % heat-inactivated fetal calf serum (BioWhittaker). Cells were first stimulated with phytohemagglutinin (20 µg/ml)-supplemented R10 (PHA P, DIFCO, Detroit, MI, USA) for three days. Then, the medium was replaced with R10 supplemented with IL2 (20 units/ml), and subsequent cultures and experiments were carried out in this medium in a 37°C humidified incubator with 5% CO₂.

Viral stocks of the TCLA X4 HIV-1_{NL} 4-3 (obtained from I. Hirsh, INSERM U 372, Marseille, France) (Adachi et al., 1986; Barre-Sinoussi et al., 1983) were produced in permissive CEM cells. HIV-1_{Hx10} and HIV-1_{MN} (obtained from Q. Sattentau) were propagated in H9 cells. Cultured supernatants from infected cells were collected at the peak of maximal viral production as assessed by p24 assay, and residual cells were removed by centrifugation at 4°C (2,000 rpm/5 min). They were sampled and stored at -80°C. The viral stock infectious titer (50 % tissue culture infectious dose, TCID₅₀) was established on C8166 cells and PBL.

### Chemical synthesis and physicochemical characterisation of synthetic MBPCs

Stepwise elongation of MBPCs was carried out on 0.1 mmol of β-Ala-Wang resin (0.38 mequiv. of amino group/g) using an automatic peptide synthesizer (Model 433A, Applied Biosystems Inc.). Trifunctional amino acids were protected on their side-chain as follows: trityl (Trt) for Gln; t-butyl (t-Bu) for Ser and Tyr, Fmoc for Lys and pentamethylchroman (Pmc) for Arg. The purity of peptides was verified by: (i) analytical reversed-phase HPLC (ii) amino acid analysis after acid hydrolysis (6 N HCl/1% phenol (mass/vol.), 20 h, 120°C, N2 atmosphere), and (iii) mass determination by matrix-assisted laser desorption ionization-time of flight (MALDI-TOF) mass spectrometry.

### HIV-1_{NL 4-3} infection of C8166 cells

Samples of 3 x 10⁵ C8166 cells were placed in 96-well plates in a volume of 100 µl of culture medium containing various concentrations of peptide. After a 1 h treatment at 37°C, 100 µl of viral solution of HIV-1_{NL4-3} were added. The cells were exposed to the virus for 1h at 37°C at a multiplicity of infection of 1,000 TCID₅₀ per ml. After thorough washing, cells were replaced in 1 ml of R10 with the treatment in 24-well plates and cultured in a 37°C incubator. C8166 culture medium was replaced at Day-4 post-infection. During this assay, treatment with peptide was permanent (before, during and after infection). Assays on C8166 cells have been performed at least twice and in duplicate. Toxicity was evaluated by daily cell count and trypan-blue exclusion assay. Infection of C8166 T-cells with HIV-1_{NL 4-3} was assessed by virus-induced cytopathic effects (syncytia formation) and by quantification of p24 viral protein in the culture supernatants. Measurements of HIV-1 p24^{gag} concentrations in the culture supernatants were achieved by ELISA, with a detection cut-off of 5 pg/ml (p24 HIV kit, NEN Dupont, Belgium; Quanti-Kine software, RILAB, Genova, Italia).

### Infection of human peripheral blood lymphocytes

Samples of 10⁶ human PBLs were placed in 96-well plates in 100 µl of R10 containing various concentrations of peptide. After 1 h treatment at 37°C, 100 µl of viral solution of HIV-1_{NL-4-3} were added. The cells were exposed to the virus for 1 h at 37°C at a multiplicity of infection of 100 TCID₅₀ per ml. After thorough washing, cells were replaced in 1 ml of culture medium in 24-well plates and cultured in a 37°C incubator flushed with 5% CO2. The PBL culture medium was replaced every 3-4 days. The cell viability was assessed by cell counts and trypan-blue exclusion assay. The viral production in the culture supernatant was quantified by p24 ELISA test, as described earlier. All the experiments have been done in blind-tests. Tests have been achieved in duplicate.

### Results

### Inhibition of HIV-1_{NL 4-3} infection of PBMCs and C8166 cells by [RQGYS]₂-K-βAla

**A).** [RQGYS]₂-K-βAla peptide (with five residues) was able to inhibit syncytium formation and p24 production of HIV-1_{NL4-3} infected C8166 cells. 100% of inhibition was obtained at a concentration of 1µM at low concentration.
**B).** [RQGYS]₂-K-βAla (at a concentration of 0.1 µM) induced a decrease (100%) in the p24 concentration in the culture supernatants from infected human PBLs.

**TABLE**

| **Inhibition of HIV-1 infection of C8166 cells by MBPCS** | | | | |
|---|---|---|---|---|
| Peptide | P24 (pg/ml) | | Syncytium formation at peptide concn of: | |
| | 1 µM | 10 µM | 1µM | 10 µM |
| 1. [RQGYSPL]₂-K-βA | 0 | 0 | - | - |
| 2. [RQGYSP]₂-K-βA | | | + | - |
| 3. [RQGYS]₂-K-βA | 0 | 0 | - | - |
| 4. [RQGY]₂-K-βA | | | ± | ± |
| 5. [RQG]₂-K-βA | | | ± | - |
| 6. AZT | 0 | 0 | - | - |
| 7. No peptide | 25000 | 23000 | ++ | ++ |

| | | | | |
|---|---|---|---|---|
| Symbols: ++ number of syncytia present in the well was similar to that in control untreated wells (35 to 40 syncytia per well), + presence of 4 to 34 syncytia in the well ± presence of 1 to 3 syncytia in the well - total absence of syncytia in the well | | | | |

**C).** [RQGY]₈-[K]₄-[K]₂-K-βA was able to inhibit syncytium formation and p24 production of HTV-1_{NL 4-3} infected C8166 cells. 100% of inhibition was obtained at a concentration of 5 µM.

The multibranched peptides were not toxic for cells, even at a concentration of 50 µM (data not shown).

## Claims

1. A multiple branch peptide construction comprising from 2 to 16 peptides each of which has the amino acid sequence RQGYS and is covalently bonded at its C-end to a core matrix, wherein the core matrix comprises:
a first lysine residue,
in the case of a construction having more than 2 peptides, two second lysine residues each bonded at its C-end to a different one of the amino groups of the first lysine residue,
in the case of a construction having more than 4 peptides, four third lysine residues each bonded at its C-end to a different one of the amino groups of the second lysine residues, and
in the case of a construction having more than 8 peptides, eight fourth lysine residues each bonded at its C-end to a different one of the amino groups of the third lysine residues.

2. A multiple branch peptide construction comprising from 8 to 16 peptides each of which has the amino acid sequence RQGY and is covalently bonded at its C-end to a core matrix, wherein the core matrix comprises:
a first lysine residue,
two second lysine residues each bonded at its C-end to a different one of the amino groups of the first lysine residue,
four third lysine residues each bonded at its C-end to a different one of the amino groups of the second lysine residues, and
in the case of a construction having more than 8 peptides, eight fourth lysine residues each bonded at its C-end to a different one of the amino groups of the third lysine residues.

3. A peptide construction according to claim 1 or claim 2 in which the peptides are replaced by synthetic constructs using the carbon skeletons of the peptides but omitting the -CONH- peptide bonds.

4. A peptide construction according to claim 1 or claim 2 in which the peptides include at least one D-amino acid residue.

5. Liposomes having an average size of greater than 150 nm, the liposomes containing a multiple branch peptide construction according to any preceding claim.

6. Liposomes according to claim 5 which have,an average size of approximately 250 nm to 400 nm.

7. Liposomes according to claim 5 or claim 6 which contain above 8% by weight of the multiple branch peptide construction.

8. A medicament comprising a multiple branch peptide construction according to any of claims 1 to 4 or liposomes according to any of claims 5 to 7 in admixture with a pharmaceutically acceptable diluent or carrier.

## Patentansprüche

1. Mehrfach verzweigtes Peptidkonstrukt, das 2 bis 16 Peptide umfasst, von denen jedes die Aminosäuresequenz RQGYS aufweist und an seinem C-Ende kovalent an eine Kernmatrix gebunden ist, wobei die Kernmatrix umfasst:
einen ersten Lysinrest,
falls ein Konstrukt mehr als 2 Peptide aufweist, zwei zweite Lysinreste, die an ihrem C-Ende jeweils an eine andere der Aminogruppen der ersten Lysinreste gebunden sind,
falls ein Konstrukt mehr als 4 Peptide aufweist, vier dritte Lysinreste, die an ihrem C-Ende jeweils an eine andere der Aminogruppen der zweiten Lysinreste gebunden sind, und
falls ein Konstrukt mehr als 8 Peptide aufweist, acht vierte Lysinreste, die an ihrem C-Ende jeweils an eine andere der Aminogruppen der dritten Lysinreste gebunden sind.

2. Mehrfach verzweigtes Peptidkonstrukt, das 8 bis 16 Peptide umfasst, von denen jedes die Aminosäuresequenz RQGY aufweist und an seinem C-Ende kovalent an eine Kernmatrix gebunden ist, wobei die Kernmatrix umfasst:
einen ersten Lysinrest,
zwei zweite Lysinreste, die an ihrem C-Ende jeweils an eine andere der Aminogruppen des ersten Lysinrests gebunden sind,
vier dritte Lysinreste, die an ihrem C-Ende jeweils an eine andere der Aminogruppen der zweiten Lysinreste gebunden sind, und
falls ein Konstrukt mehr als 8 Peptide aufweist, acht vierte Lysinreste, die an ihrem C-Ende jeweils an eine andere der Aminogruppen der dritten Lysinreste gebunden sind.

3. Peptidkonstrukt nach Anspruch 1 oder 2, in der die Peptide durch synthetische Konstrukte ersetzt sind, wobei die Kohlenstoffskelette der Peptide verwendet werden, die -CONH-Peptidbindungen jedoch fehlen.

4. Peptidkonstrukt nach Anspruch 1 oder 2, in dem die Peptide mindestens einen D-Aminosäurerest umfassen.

5. Liposomen mit einer durchschnittlichen Größe von mehr als 150 nm, wobei die Liposomen ein mehrfach verzweigtes Peptidkonstrukt nach einem der vorstehenden Ansprüche enthalten.

6. Liposomen nach Anspruch 5, die eine durchschnittliche Größe von etwa 250 nm bis 400 nm haben.

7. Liposomen nach Anspruch 5 oder 6, die mehr als 8 Gew.-% des mehrfach verzweigten Peptidkonstrukts enthalten.

8. Medikament, das ein mehrfach verzweigtes Peptidkonstrukt nach einem der Ansprüche 1 bis 4 oder Liposomen nach einem der Ansprüche 5 bis 7 im Gemisch mit einem pharmazeutisch verträglichen Verdünnungsmittel oder Träger umfasst.

## Revendications

1. Une construction peptidique à branches multiples comprenant 2 à 16 peptides, chacun ayant la séquence d'acides aminés RQGYS et lié par lien covalent à son C terminal à une matrice centrale, où la matrice centrale comprend :
un premier résidu de lysine,
dans le cas d'une construction ayant plus que 2 peptides, deux résidus de lysine secondaires, chacun lié à son C terminal à un groupe différent d'acides aminés du premier résidu de lysine,
dans le cas d'une construction ayant plus de 4 peptides, quatre résidus de lysine tertiaires, chacun lié à son C terminal à un groupe différent d'acides aminés du résidu de lysine secondaire et
dans le cas d'une construction ayant plus de 8 peptides, huit résidus de lysine quaternaires, chacun lié à son C terminal à un groupe différent d'acides aminés du résidu de lysine tertiaire.

2. Une construction peptidique à branches multiples comprenant de 8 à 16 peptides, chacun ayant la séquence d'acides aminés RQGY et lié par lien covalent au C terminal à une matrice centrale, où la matrice centrale comprend :
un premier résidu de lysine,
deux résidus de lysine secondaires, chacun lié à son C terminal à un groupe différent d'acides aminés du premier résidu de lysine,
quatre résidus de lysine tertiaires, chacun lié à son C terminal à un groupe différent d'acides aminés du résidu de lysine secondaire, et
dans le case d'une construction ayant plus de 8 peptides, huit résidus de lysine quaternaires, chacun lié à son C terminal à un groupe différent d'acides aminés du résidu de lysine tertiaire.

3. Une construction peptidique selon la revendication 1 ou la revendication 2 où les peptides sont remplacés par des constructions synthétiques utilisant l'échafaudage de carbone des peptides mais omettant les liens peptidiques -CONH-.

4. Une construction peptidique selon la revendication 1 ou la revendication 2 où les peptides comprennent au moins un résidu d'acide aminé D.

5. Les liposomes ayant une grandeur moyenne de plus de 150 nm, les liposomes contenant une construction peptidique à branches multiples selon n'importe laquelle des revendications précédentes.

6. Les liposomes selon la revendication 5 qui ont une grandeur moyenne approximative de 250 nm à 400 nm.

7. Les liposomes selon la revendication 5 et la revendication 6 qui contiennent plus que 8% en poids de la construction peptidique à branches multiples.

8. Un médicament comprenant une construction peptidique à branches multiples selon n'importe laquelle des revendications 1 à 4 ou les liposomes selon n'importe laquelle des revendications 5 à 7 dans un mélange contenant un diluant ou un porteur pharmaceutiquement acceptable.
